# EUROPEAN PATENT SPECIFICATION

(11) **EP 3 281 625 B1**
(45) Date of publication and mention of the grant of the patent: **26.02.2020**
(21) Application number: 16776569.2
(22) Date of filing: 06.04.2016
(51) Int. Cl.: A61K 9/70, A61K 31/343, A61K 31/4164, A61K 31/485, A61K 31/5575, A61K 31/558, A61K 31/59, A61K 47/10, A61K 47/14, A61K 47/32, A61K 47/34, A61K 47/36, A61K 47/38, A61K 47/40, A61K 47/42, A61K 47/46, A61P 17/04

(54) **ORAL FILM PREPARATION**
ORALES FILMPRÄPARAT
PRÉPARATION DE FILM ORAL

(30) Priority: 07.04.2015 JP 2015078297
(43) Date of publication of application: 14.02.2018
(73) Proprietor: Nipro Corporation, Osaka-shi, Osaka 531-8510 (JP)
(72) Inventor: KAWAMURA, Naohisa, Osaka-shi Osaka 531-8510 (JP); SHINKAI, Norihiro, Osaka-shi Osaka 531-8510 (JP); SHIRAISHI, Hiroaki, Osaka-shi Osaka 531-8510 (JP); TSUDA, Yo, Osaka-shi Osaka 531-8510 (JP)
(74) Representative: Vossius & Partner Patentanwälte Rechtsanwälte mbB
(86) International application number: PCT/JP2016/061271
(87) International publication number: WO 2016/163403

(56) References cited:
- EP-A1- 2 415 466
- EP-A1- 2 594 257
- WO-A1-2011/108643
- CN-A- 103 479 639
- CN-A- 105 012 276
- JP-A- S5 129 218
- JP-A- H08 282 739
- JP-A- 2005 296 381
- JP-A- 2011 511 816
- US-A1- 2005 037 055
- US-A1- 2012 328 675
- US-A1- 2014 070 440
- US-A1- 2015 051 408
- EVA MARIA HOFFMANN ET AL: "Advances in orodispersible films for drug delivery", EXPERT OPINION ON DRUG DELI, INFORMA HEALTHCARE, GB, vol. 8, no. 3, 1 March 2011 (2011-03-01), pages 299-316, XP008157100, ISSN: 1742-5247, DOI: 10.1517/17425247.2011.553217
- Jnanadeva Bhat M,: "Role of packaging material on pharmaceutical product stability", , XP002783038, Retrieved from the Internet: URL:http://www.ipapharma.org/events/stabil ity/jbhat.pdf [retrieved on 2018-07-13]

## Description

### TECHNICAL FIELD

The present invention relates to an oral film preparation for medical use, in particular to an orally disintegrating film comprising nalfurafine hydrochloride as an active ingredient and being excellent in content uniformity.

### BACKGROUND ART

Generally granules, grains, powders, tablets, capsules, and the like are known as solid preparations of high potency drugs, for example, nalfurafine hydrochloride, and the like. However, in these dosage forms, since a single dose of the high potency drug is very small, the content thereof per unit preparation is very low, and it is difficult to produce a preparation being excellent in content uniformity.

Patent Document 1 discloses a sheet-like solid drug composition characterized in that the composition is prepared by printing, coating, spraying or injecting a solution or suspension containing a substance having a physiological activation action in a trace amount into a pharmaceutically acceptable sheet-like carrier for the purpose of solving a problem with generation of dusts in a preparation process of a substance having a physiological activation action in a trace amount and an adverse effect thereof on workers and environmental pollution thereby.

On the other hand, an oral film preparation, especially an oral disintegrating type film preparation has been developed in recent years as one of dosage forms in a pharmaceutical field from the viewpoint of an advantage that it can be taken without water, and portability is satisfactory. However, in the production of an oral film preparation, properties thereof such as brittleness, adhesiveness, and hygroscopicity, and a problem of a lack of uniformity in the interior of the dosage form are recognized (Patent Document 2), and application of an oral film preparation to high potency drugs and evaluation of content uniformity are not known.

US 2014/ 0070441 A1 and US 2005/0037055 A1 describe drug delivery systems made from a film containing polyethylene oxide. EP 2594257 A1 discloses orodispersible films. US 2015/0051408 A1 deals with packaged products of a solid preparation containing 5-hydroxy-1H-imidazole-4-carboxamide. US 2012/0328675 A1 describes a film preparation containing a medicament with unpleasant taste. An orally disintegrating coated tablet is described in EP 2415466 A1. Expert Opin. Drug Deliv. (2011) 8(3) pages 299-316 deals with advances in orodispersible films for drug delivery.

### PRIOR ART DOCUMENTS

Patent Document 1: JP H05-124954 A
Patent Document 2: JP 2013-527164 A

### SUMMARY OF THE INVENTION

### PROBLEM TO BE SOLVED BY THE INVENTION

In Patent Document 1, a problem with respect to content uniformity is not pointed out and in an application to a carrier by printing, or the like, it is difficult to secure satisfactory content uniformity, and there is room for improvement.

Therefore, an object of the present invention is to provide an oral film preparation comprising a high potency drug and being excellent in content uniformity, a simple method for producing the film preparation, and a package comprising the oral film preparation and being excellent in stability of the oral film preparation.

### MEANS TO SOLVE THE PROBLEM

The inventors of the present invention have made intensive studies in the light of the above-mentioned problems, and as a result, have found that a high potency drug can be contained in a low content uniformly in an oral film preparation obtained by bringing the high potency drug and a film-forming polymer into a solution state, and spreading and then drying the solution, and have completed the present invention.

Namely, the present invention relates to:
[1] an oral film preparation obtained by spreading a solution comprising a high potency drug and a film-forming polymer, and then drying the solution,
   wherein the high potency drug is nalfurafine hydrochloride, wherein the film-forming polymer is a mixture of hydroxypropyl cellulose and hydroxypropyl methyl cellulose, and wherein the preparation further comprises polyethylene glycol as a plasticizer;
[2] the oral film preparation of the above [1], further comprising an antioxidant,
[3] the oral film preparation of any of the above [1] to [2], wherein the oral film preparation is an orally disintegrating film,
[4] a package including the oral film preparation of any of the above [1] to [3], the package being obtained by:
   (a) packaging the oral film preparation in a packaging material and after nitrogen substitution, sealing the packaging material, and/or
   (b) sealing the oral film preparation together with a deoxidant in a packaging material,
[5] a package obtained by packaging the oral film preparation of any of the above [1] to [3] with a packaging material having a deoxidizing function,
[6] the package of the above [4] or [5], wherein a dehumidifying agent is further sealed, and
[7] a method for producing an oral film preparation comprising a high potency drug, which comprises spreading a solution containing the high potency drug, a film-forming polymer and a plasticizer and drying the solution, wherein the high potency drug, the film-forming polymer and the plasticizer are as defined in [1] above.

### EFFECTS OF THE INVENTION

According to the present invention, by spreading a solution containing a high potency drug, a film-forming polymer and a plasticizer and then drying the solution, an oral film preparation being excellent in content uniformity of the high potency drug can be provided. From a viewpoint that the oral film preparation can be easily taken without water, the medication compliance can be improved. Further, it is a big advantage for a patient restricted to take water in a limited amount such as a dialysis patient that the administration can be made without water. Furthermore, according to the present invention, stability of the high potency drug can be improved by a package obtained by (a) packaging the oral film preparation in a packaging material and after substitution with nitrogen, sealing the packaging material, and/or (b) sealing the oral film preparation together with a deoxidant in a packaging material, or packaging the oral film preparation with a packaging material having a deoxidizing function. Furthermore, according to the present invention, by spreading a solution containing a high potency drug, a film-forming polymer and a plasticizer and then drying the solution, an oral film preparation being excellent in the content uniformity of the high potency drug can be produced by a very simple method.

### BRIEF DESCRIPTION OF THE DRAWINGS

FIG. 1 is a graph showing stability of nalfurafine hydrochloride at a storage temperature of 80°C.
FIG. 2 is a graph showing stability of nalfurafine hydrochloride at a storage temperature of 60°C.
FIG. 3 is a graph showing stability of nalfurafine hydrochloride at a storage temperature of 60°C.

### EMBODIMENT FOR CARRYING OUT THE INVENTION

The present invention relates to an oral film preparation comprising a high potency drug as an active ingredient, and is characterized in that the oral film preparation is obtained by spreading a solution containing a high potency drug, a film-forming polymer and a plasticizer into a film shape and then drying the solution.

In the present invention, the high potency drug means a drug contained in an amount of 0.1 mg or less per one film; the drug is nalfurafine hydrochloride.

Nalfurafine hydrochloride is a morphinan compound having a chemical name: (2E)-N-[(5R,6R)-17-(cyclopropylmethyl)-4,5-epoxy-3,14-dihydroxymorp hinan-6-yl]-3-(furan-3-yl)-N-methylprop-2-enamide monohydrochloride. Nalfurafine hydrochloride is a selective κ-opioid receptor agonist, and exhibits an effect on improvement of pruritus in hemodialysis patients. Currently, nalfurafine hydrochloride is available on the market as REMITCH (registered trademark) CAPSULES 2.5 µg, which is an oral preparation of a soft capsule dosage form. Therefore, it is very beneficial for a patient having a water intake restriction such as a hemodialysis patient that the administration of nalfurafine hydrochloride can be made without water, and since the effect of the present invention can be demonstrated, in the present invention, it is preferable to use nalfurafine hydrochloride as a high potency drug.

The content of the high potency drug can be easily set by a person skilled in the art, depending on kind and physical properties of an active drug to be used, and, for example, when a nalfurafine hydrochloride is used, usually it is contained in an amount of preferably 0.1 to 80 µg, more preferably 0.5 to 40 µg per one sheet of the film preparation (one dose). When the content of nalfurafine hydrochloride per one sheet of the film preparation exceeds 80 µg, serious side effects tend to develop, and when less than 0.1 µg, there is a tendency that a sufficient medicinal effect cannot be exhibited.

It is necessary to allow the oral film preparation to contain a film-forming polymer (base material) and a plasticizer other than the high potency drug being an active ingredient. Also, if necessary, various additives which are commonly used in the art, i.e. a surfactant, a stabilizer, a thickener, an antiseptic agent, an antioxidant, a pH regulator, a die, a pigment, a perfume, a saccharide, a disintegrating agent, an excipient, a flavoring agent, an essential oil, a binder, a moisture-proof agent and the like can be contained within a range not to impair the effects of the present invention.

The film-forming polymer is a mixture of hydroxypropyl cellulose (HPC), and hypromellose (hydroxypropyl methyl cellulose) (HPMC).

The content of the film-forming polymer is not particularly limited, and is preferably 30% by mass or more, more preferably 40% by mass or more in the oral film preparation. When the content of the film-forming polymer is less than 30 parts by mass, there is a tendency that the film becomes brittle, thereby causing a problem with the film quality.

As plasticizer polyethylene glycol is used.

When the plasticizer is contained in the oral film preparation, the content thereof is not particularly limited, and is preferably 40% by mass or less, more preferably 30% by mass or less. When the content of the plasticizer exceeds 40% by mass, there is a tendency that sufficient strength cannot be maintained and thereby a film cannot be formed. The content of the plasticizer in the oral film preparation is preferably 0.1% by mass or more, more preferably 0.5% by mass or more. When the content of the plasticizer is less than 0.1% by mass, there is a possibility that flexibility of the preparation is not obtained, which has an adverse effect on quality thereof.

Examples of the antioxidant include ascorbic acid, bisulfite, sulfite, dibutylhydroxytoluene (BHT), natural vitamin E, tocopherol, d-delta-tocopherol, tocopherol acetate, concentrated mixed tocopherol, thiosulfate, propyl gallate, pyrosulfite, nitrite, L-ascorbyl stearate, α-thioglycerol, edetate, erythorbic acid, cysteine hydrochloride, citrate, dichloroisocyanurate, soya lecithin, thioglycolate, thiomalate, ascorbyl palmitate, butylhydroxyanisole, 1,3-butylene glycol, benzotriazole, pentaerythrityl-tetrakis[3-(3,5-di-t-butyl-4-hydroxyphenyl)propionate], 2-mercaptobenzimidazole, and the like.

When the antioxidant is contained in the oral film preparation, the content thereof is not particularly limited, and is preferably 70% by mass or less, more preferably 60% by mass or less. When the content of the antioxidant exceeds 70% by mass, a flavor is impaired due to taste and odor peculiar to the antioxidant, and there is a case where quality of the preparation is deteriorated. The content of the antioxidant in the oral film preparation is preferably 0.0001% by mass or more, more preferably 0.001% by mass or more, further preferably 0.01% by mass or more. When the content of the antioxidant is less than 0.0001% by mass, there is a possibility that a sufficient antioxidation effect cannot be obtained, which has an adverse effect on quality thereof.

Particularly in terms of content uniformity, the oral film preparation according to the present invention can be produced by applying a uniform solution including a film-forming polymer, a plasticizer and active ingredients in a predetermined thickness and then drying the solution. More specifically, an oral film preparation having a thickness, for example, within a range of 5 to 400 µm, preferably within a range of 10 to 300 µm can be obtained by (1) dissolving the film-forming polymer and the additives such as a plasticizer in a mixed solvent of water and an alcohol to obtain a solution, (2) dissolving a drug as an active ingredient in water, (3) kneading the solutions of (1) and (2) to obtain a drug solution, and (4) spreading the obtained drug solution in a predetermined thickness and then drying the solution.

In the present invention, the oral film preparation can be easily taken without water, and therefore, is preferably an orally disintegrating film from the viewpoint that an improvement of medication compliance by prevention of erroneous swallowing by a dysphagia patient can be expected, and that moisture management for a dialysis patient having a water intake restriction can be expected to be easily performed. The orally disintegrating film can be produced by appropriately combining the film forming polymer and the plasticizer described above, i.e. hydroxypropyl cellulose and hypromellose as suitable film-forming polymers and polyethylene glycol as a plasticizer.

In the present invention, in the case of using a drug to be affected by oxidation, especially nalfurafine hydrochloride as an active ingredient, it was confirmed that by taking deoxidation means, the degradation of the active ingredient can be reduced and the stable preparation can be obtained.

Examples of such a deoxidizing means include a method for allowing an antioxidant to be contained in the above-mentioned oral film preparation, a method for conducting substitution with nitrogen at the time of packaging, a method for sealing a deoxidant in the package, and a method of using a packaging material, for example, a packaging film having a deoxidizing function, and the method of using a packaging material having a deoxidizing function is preferable from the viewpoint of easy extraction of a preparation from a packaging material, cost reduction of starting materials, and simplification of a manufacturing process.

Examples of the deoxidant allowed to coexist in the package include inorganic deoxidants such as iron and cerium oxide, organic deoxidants and the like, and one having a flat form such as a sheet-like or a stick-like shape similar to the oral film preparation of the present invention is preferable. Such a deoxidant is not particularly limited, and there are commercially available deoxidants usable for pharmaceutical applications, for example, "PharmaKeep" manufactured by Mitsubishi Gas Chemical Company, Inc., and "AGELESS" manufactured by Mitsubishi Gas Chemical Company, Inc.

The packaging material having a deoxidizing function is not particularly limited, and a packaging film including a deoxidant is preferable. Such a packaging film can be classified into one including an inorganic deoxidant such as iron or cerium oxide, or an organic deoxidant-containing film. These films are not particularly limited, and examples thereof include commercially available films that can be used for pharmaceutical applications, and for example, "OxyCatch (registered trademark)" manufactured by Kyodo Printing Co. Ltd. and "High Star O2" manufactured by Starplastic Industry Inc. can be used as a packaging material containing cerium oxide, and "AGELESS OMAC (registered trademark)" manufactured by Mitsubishi Gas Chemical Company, Inc. and "Oxyguard" manufactured by Toyo Seikan Co., Ltd. can be used as a packaging material containing iron.

Furthermore, in the oral film preparation of the present invention, even when the drug in the film cannot be kept sufficiently stable with only a deoxidizing means, there is a case where the drug in the film can be stably maintained by combination use of a dehumidifying agent. In such a case, combination use of the dehumidifying agent is preferable. It is a matter of course to allow a dehumidifying agent having only a dehumidifying function to coexist with the preparation in the package which is made by packaging with a packaging material having a deoxidizing function, and a dehumidifying agent commonly used in the field of pharmaceuticals can be used as such a dehumidifying agent.

In yet another embodiment, the present invention relates to a method for producing an oral film preparation containing a high potency drug. The method for producing an oral film preparation containing a high potency drug of the present invention is characterized by using a solution containing a film-forming polymer in which a high potency drug is dissolved and a plasticizer, and comprises spreading and drying the solution. For example, as described above for the oral film preparation of the present invention, the producing method of the present invention can be performed specifically by a step 1 of dissolving the film-forming polymer and optionally other additives in a solvent such as water; a step 2 of dissolving the high potency drug in a solvent such as water; a step 3 of obtaining a solution containing the high potency drug and the film-forming polymer by kneading the solution obtained in the step 1 and the solution obtained in the step 2; a step 4 of spreading the solution obtained in the step 3 on a liner or the like in a predetermined thickness if necessary; a step 5 of drying the spread solution; and a step 6 of cutting the dried film to an appropriate size; and the like.

The above description made for the "oral film preparation" is also applied similarly to the "method for producing an oral film preparation containing a high potency drug" unless otherwise contradictory, and further, the above description made for the "method for producing an oral film preparation containing a high potency drug" is also applied similarly to the above-mentioned "oral film preparation".

The present invention is described in more detail by means of Examples and Comparative Examples, but the present invention is not limited to these Examples.

### EXAMPLE

Ingredients used in Examples and Comparative Examples are those described in Japanese Pharmacopoeia or Japanese Pharmaceutical Excipients.

### Examples 1 to 4

According to the formulation in Table 1, (a) hydroxypropyl cellulose, hypromellose and polyethylene glycol were added to a mixed solution of water and ethanol, followed by stirring at room temperature for 30 minutes to be dissolved in the solution (Solution A); (b) nalfurafine hydrochloride was added to an appropriate amount of water (except one in Table 1), followed by stirring at room temperature for 5 minutes to be dissolved in the water (Solution B); and (c) the Solution A of (a) and the Solution B of (b) were mixed and the mixture was kneaded at room temperature for 30 minutes using a three-one motor to obtain a Drug Solution C.

The obtained Drug Solution C was coated uniformly on a liner so that the coating thickness after drying became 40 µm, followed by drying at 70°C for 10 minutes in Example 1, at 80°C for 10 minutes in Example 2, at 80°C for 15 minutes in Example 3, and at 80°C for 30 minutes in Example 4. The obtained films were punched into a size of 1.5 × 2.0 cm (3.0 cm²) to obtain film preparations. A theoretical mass of the obtained film preparation per sheet was 12 mg, and the drug content was 2.5 µg.

**Table 1**

| Composition | Charged amount (g) | Dry mass % |
|---|---|---|
| Nalfurafine hydrochloride | 0.002083 | 0.02083 |
| HPC | 4.499 | 44.98959 |
| HPMC | 4.499 | 44.98959 |
| PEG400 | 1.00 | 10.00 |
| Ethanol | 28.33 | 0.00 |
| Water | 28.33 | 0.00 |

### Example 5

According to the formulation in Table 2, (a) hydroxypropyl cellulose, hypromellose, polyethylene glycol and an antioxidant (sodium sulfite) were added to a mixed solution of water and ethanol, followed by stirring at room temperature for 30 minutes to be dissolved in the solution (Solution A); (b) nalfurafine hydrochloride was added to an appropriate amount of water (except one in Table 2), followed by stirring at room temperature for 5 minutes to be dissolved in the water (Solution B); and (c) the Solution A of (a) and the Solution B of (b) were mixed and the mixture was kneaded at room temperature for 30 minutes using a three-one motor to obtain a Drug Solution C.

The obtained Drug Solution C was coated uniformly on a liner so that the coating thickness after drying became 40 µm, followed by drying at 80°C for 15 minutes. The obtained film was punched into a size of 1.5 × 2.0 cm (3.0 cm²) to obtain a film preparation. A theoretical mass of the obtained film preparation per sheet was 12 mg, and the drug content was 2.5 µg.

**Table 2**

| Composition | Charged amount (g) | Dry mass % |
|---|---|---|
| Nalfurafine hydrochloride | 0.002083 | 0.02083 |
| HPC | 4.399 | 43.98959 |
| HPMC | 4.399 | 43.98959 |
| PEG400 | 1.00 | 10.00 |
| Sodium sulfite | 0.20 | 2.00 |
| Ethanol | 28.33 | 0.00 |
| Water | 28.33 | 0.00 |

### Test Example 1 (Content uniformity test)

For the film preparations obtained in Examples 1 to 4 (n=3 in each of Examples), the concentrations of the drug of the three sheets of film preparations per one lot were measured to calculate an average value and a standard deviation. A relative standard deviation was calculated as an index for the content uniformity from the calculated average value and standard deviation. The results are shown in Table 3.

**Table 3**

| Example | Actual value (%) | Concentration (%) | RSD |
|---|---|---|---|
| 1 | 102.6 | 101.9 | 0.32 |
| 2 | 102.6 | 103.5 | 1.97 |
| 3 | 103.4 | 102.8 | 2.19 |
| 4 | 104.2 | 103.2 | 0.61 |

From Table 3, it is seen that in any of Examples, the relative standard deviation is less than 2.2 and the content uniformity is excellent.

### Test Example 2 (Stability test)

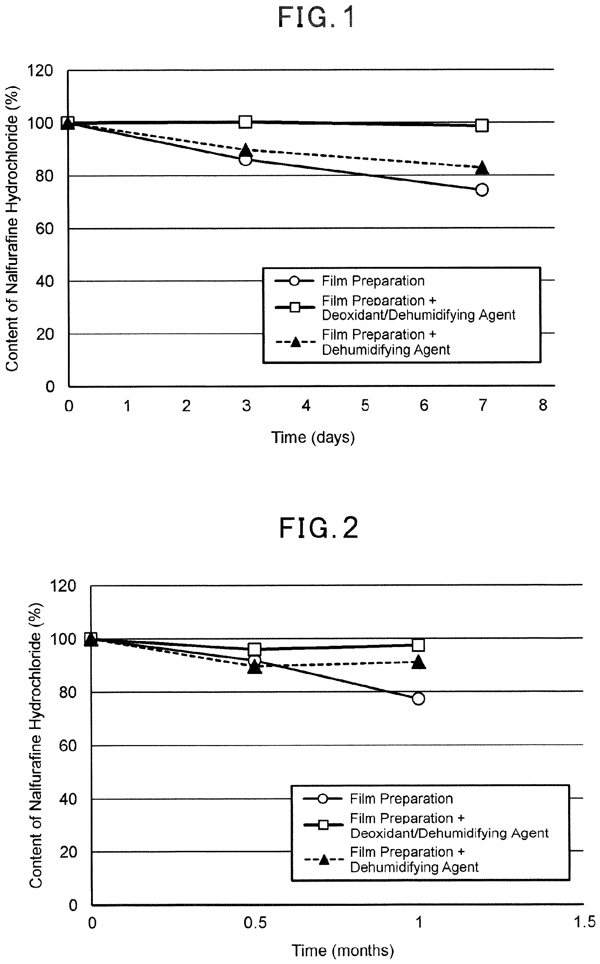

A film preparation produced in the same manner as in Example 3 was sealed as it was or together with a deoxidant/dehumidifying agent (PharmaKeep (KD-20) manufactured by Mitsubishi Gas Chemical Company, Inc.) in Easy Peel (manufactured by Toppan Printing Co., Ltd.), or sealed in a packaging material having a dehumidifying function (Moisture Guard (MG) manufactured by Toyo Seikan Co., Ltd.), followed by storing in a thermostat of 80°C. The content of the drug (nalfurafine hydrochloride) in the preparation was measured when starting the storing and during the storing (three days after and seven days after). The results are shown in FIG. 1 as a drug content (%) assuming that the drug content when starting the storing is 100.

### Test Example 3 (Stability test)

The content of the drug (nalfurafine hydrochloride) was measured in the same manner as in Test Example 2 except that the storing temperature was changed to 60°C and during the storing, measurement was made 0.5 month after and one month after. The results are shown in FIG. 2 as a drug content (%) assuming that the drug content when starting the storing is 100.

### Test Example 4 (Stability test)

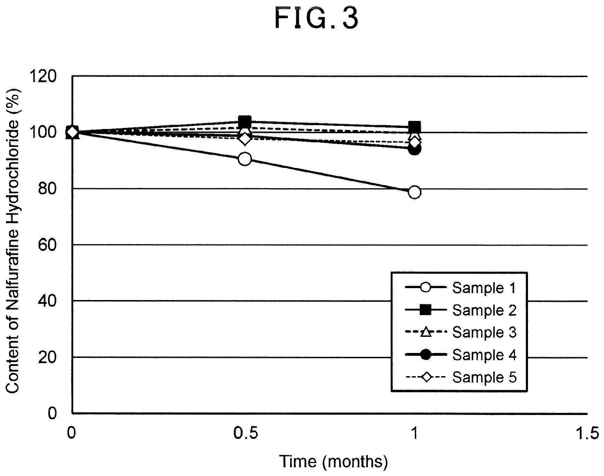

The film preparation was sealed in a packaging material shown in Table 4, and stored in a thermostat of 60°C. Film preparations produced in the same manner as in Example 3 were used as Samples 1 to 4, and the film preparation produced in Example 5 was used as Sample 5. The contents of the drug (nalfurafine hydrochloride) in the preparation were measured when starting the storing and during the storing (0.5 month after and one month after). The results are shown in FIG. 3 as a drug content (%) assuming that the drug content when starting the storing is 100.

**Table 4**

| Sample No. | Packaging material (front) | Packaging material (back) | Antioxidant |
|---|---|---|---|
| 1 | Easy Peel ^{*1} | Easy Peel | - |
| 2 | OxyCatch ^{*2} | Easy Peel | - |
| 3 | OxyCatch | OxyCatch | - |
| 4 | AGELESS OMAC ^{*3} | AGELESS OMAC | - |
| 5 | Easy Peel | Easy Peel | 2% Sodium sulfite |

| | | | |
|---|---|---|---|
| *1 Packaging material manufactured by Toppan Printing Co., Ltd. *2 Packaging material including cerium oxide manufactured by Kyodo Printing Co., Ltd. *3 Packaging material including iron manufactured by Mitsubishi Gas Chemical Company, Inc. | | | |

It is seen from FIGS. 1 to 3 that the oral film preparation of the present invention assures that stability of the drug can be maintained even in the case of a long-term storage thereof by taking a deoxidizing means.

## Claims

1. An oral film preparation obtainable by spreading a solution containing a high potency drug and a film-forming polymer and drying the solution,
wherein the high potency drug is nalfurafine hydrochloride,
wherein the film-forming polymer is a mixture of hydroxypropyl cellulose and hydroxypropyl methyl cellulose, and
wherein the preparation further comprises polyethylene glycol as a plasticizer.

2. The oral film preparation of claim 1, further comprising an antioxidant.

3. The oral film preparation of any one of claims 1 to 2, wherein the oral film preparation is an orally disintegrating film.

4. A package including the oral film preparation of any one of claims 1 to 3, the package being obtainable by
(a) packaging the oral film preparation in a packaging material and after nitrogen substitution, sealing the packaging material, and/or
(b) sealing the oral film preparation together with a deoxidant in a packaging material.

5. A package obtainable by packaging the oral film preparation of any one of claims 1 to 3 with a packaging material having a deoxidizing function.

6. The package of claim 4 or 5, wherein a dehumidifying agent is further sealed.

7. A method for producing an oral film preparation comprising a high potency drug, which comprises spreading a solution containing the high potency drug, a film-forming polymer and a plasticizer and drying the solution;
wherein the high potency drug is nalfurafine hydrochloride, the film-forming polymer is a mixture of hydroxypropyl cellulose and hydroxypropyl methyl cellulose, and the plasticizer is polyethylene glycol.

## Patentansprüche

1. Ein orales Filmpräparat, erhältlich durch Auftragen einer Lösung, die einen hochwirksamen Wirkstoff und ein filmbildendes Polymer enthält, und Trocknen der Lösung,
wobei es sich bei dem hochwirksamen Wirkstoff um Nalfurafin-Hydrochlorid handelt,
wobei das filmbildende Polymer ein Gemisch aus Hydroxypropylcellulose und Hydroxypropylmethylcellulose ist, und
wobei das Präparat ferner Polyethylenglycol als einen Weichmacher umfasst.

2. Das orale Filmpräparat nach Anspruch 1, ferner umfassend ein Antioxidationsmittel.

3. Das orale Filmpräparat nach einem der Ansprüche 1 bis 2, wobei das orale Filmpräparat ein sich im Mund auflösender Film ist.

4. Eine Verpackung, welche das orale Filmpräparat nach einem der Ansprüche 1 bis 3 enthält, wobei die Verpackung erhältlich ist durch
(a) Verpacken des oralen Filmpräparats in einem Verpackungsmaterial und, nach Stickstoffsubstitution, Verschließen des Verpackungsmaterials, und/oder
(b) Einschließen des oralen Filmpräparats zusammen mit einem desoxidierenden Mittel in einem Verpackungsmaterial.

5. Eine Verpackung, erhältlich durch Verpacken des oralen Filmpräparats nach einem der Ansprüche 1 bis 3 mit einem Verpackungsmaterial, das eine desoxidierende Funktion hat.

6. Die Verpackung nach Anspruch 4 oder 5, wobei ein entfeuchtendes Mittel ferner eingeschlossen wird.

7. Ein Verfahren zur Herstellung eines oralen Filmpräparats, welches einen hochwirksamen Wirkstoff umfasst, umfassend Auftragen einer Lösung, die den hochwirksamen Wirkstoff, ein filmbildendes Polymer und einen Weichmacher enthält, und Trocknen der Lösung;
wobei der hochwirksame Wirkstoff Nalfurafin-Hydrochlorid ist, das filmbildende Polymer ein Gemisch aus Hydroxypropylcellulose und Hydroxypropylmethylcellulose ist und der Weichmacher Polyethylenglycol ist.

## Revendications

1. Préparation de film oral pouvant être obtenue par étalement d'une solution contenant un médicament à forte puissance et un polymère filmogène et séchage de la solution,
dans laquelle le médicament à forte puissance est le chlorhydrate de nalfurafine,
dans laquelle le polymère filmogène est un mélange d'hydroxypropyl cellulose et d'hydroxypropyl méthyl cellulose, et
dans laquelle la préparation comprend en outre du polyéthylène glycol en tant que plastifiant.

2. Préparation de film oral selon la revendication 1, comprenant en outre un anti-oxydant.

3. Préparation de film oral selon l'une quelconque des revendications 1 et 2, dans laquelle la préparation de film oral est un film à désintégration orale.

4. Conditionnement comprenant la préparation de film oral selon l'une quelconque des revendications 1 à 3, le conditionnement pouvant être obtenu par
(a) conditionnement de la préparation de film oral dans un matériau de conditionnement et après substitution avec de l'azote, scellement étanche du matériau de conditionnement, et/ou
(b) scellement étanche de la préparation de film oral conjointement avec un désoxydant dans un matériau de conditionnement.

5. Conditionnement pouvant être obtenu par conditionnement de la préparation de film oral selon l'une quelconque des revendications 1 à 3 avec un matériau de conditionnement ayant une fonction désoxydante.

6. Conditionnement selon l'une des revendications 4 ou 5, dans lequel un agent de déshumidification est en outre scellé.

7. Procédé de production d'une préparation de film oral comprenant un médicament à forte puissance, qui comprend l'étalement d'une solution contenant le médicament à forte puissance, un polymère filmogène et un plastifiant et le séchage de la solution ;
dans lequel le médicament à forte puissance est le chlorhydrate de nalfurafine, le polymère filmogène est un mélange d'hydroxypropyl cellulose et d'hydroxypropyl méthyl cellulose, et le plastifiant est le polyéthylène glycol.
